# EUROPEAN PATENT APPLICATION

(11) **EP 4 212 913 A1**
(43) Date of publication of application: **19.07.2023**
(21) Application number: 21866812.7
(22) Date of filing: 09.09.2021
(51) Int. Cl.: G01T 1/161, G01T 1/164, G01T 1/20

(54) **SIGNAL PROCESSING SYSTEM, POSITRON EMISSION TOMOGRAPHY DEVICE, AND POSITRON EMISSION TOMOGRAPHY METHOD**

(30) Priority: 09.09.2020 JP 2020151581
(71) Applicant: Mitsubishi Chemical Corporation, Chiyoda-ku Tokyo 100-8251 (JP); Tohoku University, Sendai-shi, Miyagi 980-8577 (JP)
(72) Inventor: HAZU, Koji, Tokyo 100-8251 (JP); HORIBE, Kentaro, Tokyo 100-8251 (JP); YAMAHARA, Keiji, Tokyo 100-8251 (JP); KUROSAWA, Shunsuke, Sendai-shi, Miyagi 980-8577 (JP); YOSHIKAWA, Akira, Sendai-shi, Miyagi 980-8577 (JP)
(74) Representative: Hoffmann Eitle
(86) International application number: PCT/JP2021/033091
(87) International publication number: WO 2022/054854

(57) **Abstract**

Provided are a signal processing system, a positron emission tomography device, and a positron emission tomography method that improve detection efficiency, further decrease a total amount of radiation by the improvement of detection efficiency, and consequently improve an S/N value by decreasing random coincidences, compared with conventional systems, devices, and methods. The signal processing system generates image data, based on an electric signal group output from a radiation detector, and recognizes the electric signal group as a processing target, and the electric signal group includes at least part of an electric signal group meeting the following requirements: the electric signal group is an electric signal group with a signal value within a predetermined range, the electric signal group corresponding to a gamma ray with energy equal to or less than 375 keV; the predetermined range is equal to or greater than 50% and equal to or less than 80% relative to a 100% signal value; and the 100% signal value is a signal value detected when a gamma ray with energy of 511 keV enters a radiation detection element in the radiation detector and is totally absorbed by the radiation detection element.

## Description

### TECHNICAL FIELD

The present invention relates to a positron emission tomography (PET) device used for a high-count radiation detection device such as a positron emission tomography device.

### BACKGROUND ART

A positron emission tomography device measuring high-energy radiation such as a gamma ray is used in a positron tomography (PET) or the like and, for example, is used in a diagnostic device predicting a position of a cancerous cell by injecting a substance (tracer) acquired by substituting a positron emission nuclide (radiation source) for part of elements in a molecule localized in a cancerous cell into a patient, measuring radiation caused by the tracer in the patient body, and determining the source position in nuclear medicine.

In general, a positron emission tomography device includes a scintillator section including a scintillator receiving radiation and emitting an electromagnetic wave such as visible light, a conversion-output section receiving an electromagnetic wave emitted by the scintillator, converting the received electromagnetic wave into an electric signal, and outputting the resulting signal, and a signal processing system converting the electric signal output by the conversion-output section into image data.

In the positron emission tomography device, by using a phenomenon that two radiations are emitted simultaneously in directions opposite to each other as a result of electron-positron pair annihilation in the tracer or the like in a subject, when the radiations enter the scintillators at two separate positions almost simultaneously (within a predetermined time span) and are detected, it is assumed that "two radiations are generated at a midpoint of a line connecting the positions of the two scintillators in accordance with the principle," and the position of the radiation source is calculated from the positions of the scintillators (the detection method may be hereinafter expressed as a "coincidence counting" method, and the number of times coincidences occur may be referred to as "the number of coincident events"). Furthermore, by recording a radiation generation frequency for each position where radiation is estimated to be generated, a relative concentration density of radiation sources in the subject can be measured, and an image can be synthesized from the information. Note that the predetermined time span may be referred to as a "time window."

Various types of noise that appear to be simultaneous coefficients other than a coincidence caused by electron-positron pair annihilation occurring near a tracer (may also be expressed as a true coincidence) are generated in the positron emission tomography device. Examples of noise include radiation generated by the scintillator itself (may be hereinafter described as "inherent background radiation"), background radiation composed of radiation generated inside and/or outside the device, "back scattering" caused by radiation being a measurement target entering the scintillator after being scattered by a wall in the device, a "random coincidence" caused by two radiations generated at separate locations randomly and simultaneously entering different scintillators, a "scattered coincidence" caused by either of two radiations simultaneously generated from the same radiation source in opposite directions causing Compton scattering and changing the angle and energy. These types of noise hinder accurate measurement and therefore need to be suitably excluded. However, despite inclusion of uncertainty about positional information, a scattered coincidence may be used as a signal.

Therefore, a method for accurately acquiring positional information of a radiation source by improving the ratio of a signal to noise (S/N value) has been reported. Further, a mechanism for reducing an amount of exposure of a subject by improving the number of events per total amount of radiation has been reported.

For example, Non-Patent Document 1 describes development of a circuit automatically performing an operation of, when emitted light corresponding to energy equal to or less than a specific threshold value is detected by a scintillator, determining the light to be noise and not performing data collection, on the basis of energy of inherent background radiation (a gamma ray), a gamma ray entering by back scattering, and a gamma ray generated in a scattered coincidence event being different from energy of a gamma ray in a true coincidence (511 keV).

### RELATED ART DOCUMENT

### NON-PATENT DOCUMENT

[Non-Patent Document 1] IEEE TRANSACTIONS ON NUCLEAR SCIENCE, VOL. 63 (2016), pages 1327 to 1334

### SUMMARY OF THE INVENTION

### PROBLEMS TO BE SOLVED BY THE INVENTION

However, in the previously reported simultaneous measurement devices, the signal processing system eliminates, as noise, a value very close to a signal value of an electric signal corresponding to energy initially possessed by radiation originating from a radiation source (may also be hereinafter described as initial energy), such as an electric signal corresponding to energy equal to or less than around 500 keV when initial energy is 511 keV.

Therefore, a scattered coincidence energy of which is decreased by Compton scattering may not be used as a signal, and there is an issue that as the number of events generated according to an amount of radiation decreases, a total amount of radiation needs to be increased for acquiring a clear image, an amount of exposure of a subject increases, and an S/N value may be rapidly degraded due to rapid increase in random coincidences when the total amount of radiation exceeds a certain value.

In addition, when a time window is set wide in order to improve the number of events, there is an issue that a random coincidence cannot be suitably excluded, and therefore the S/N value is degraded.

Then, an object of the present invention is to provide a signal processing system, a positron emission tomography device, and a positron emission tomography method that improve detection efficiency, decrease a total amount of radiation by the improvement of detection efficiency, and consequently improve an S/N value by decreasing random coincidences, compared with conventional systems, devices, and methods.

### MEANS FOR SOLVING THE PROBLEMS

As a result of intensive studies in view of the aforementioned issues, the present inventors have discovered that use of a signal processing system including, in processing targets thereof, at least part of electric signals with signal values within a predetermined range or electric signals corresponding to a gamma ray with energy in a predetermined range can increase the number of events per generated amount of radiation and decrease an amount of exposure of a subject, thereby completing the present invention.

Specifically, the spirit and scope of the present invention include the following.
[1] A signal processing system generating image data, based on an electric signal group output from a radiation detector,
   wherein the signal processing system recognizes the electric signal group as a processing target, and
   the electric signal group includes at least part of an electric signal group meeting requirements described below:
      the electric signal group is an electric signal group with a signal value within a predetermined range, the electric signal group corresponding to a gamma ray with energy equal to or less than 375 keV;
      the predetermined range is equal to or greater than 50% and equal to or less than 80% relative to a 100% signal value; and
      the 100% signal value is a signal value detected when a gamma ray with energy of 511 keV enters a radiation detection element in a radiation detector and is totally absorbed by the radiation detection element.
[2] A signal processing system generating image data, based on an electric signal group output from a radiation detector,
   wherein the signal processing system recognizes the electric signal group as a processing target, and
   the electric signal group includes at least part of an electric signal group corresponding to a gamma ray with an energy value within a predetermined range, and the predetermined range is equal to or greater than 232 keV and equal to or less than 340 keV.
[3] A positron emission tomography device including the signal processing system according to [1] or [2] and a radiation detector section.
[4] The positron emission tomography device according to [3], wherein the radiation detector section includes components described below:
   a scintillator section including a scintillator receiving radiation and emitting an electromagnetic wave; and a conversion-output section receiving an electromagnetic wave emitted from the scintillator, converting the received electromagnetic wave into a pulse-shaped electric signal, and outputting the resulting signal.
[5] The positron emission tomography device according to [4], wherein the scintillator meets a characteristic described below:
   intensity of inherent background of the scintillator is equal to or less than 200 Hz/cm³ in a range of a signal value equal to or greater than 10% and equal to or less than 120% with a signal value of the pulse-shaped electric signal when a gamma ray with energy of 511 keV enters the scintillator and is totally absorbed by the scintillator as 100%.
[6] The positron emission tomography device according to [4] or [5], wherein a time window in the conversion-output section is equal to or less than 180 ns.
[7] The positron emission tomography device according to any one of [4] to [6], wherein a fluorescence decay time (DT) of the scintillator when the scintillator is irradiated with a gamma ray is equal to or less than 25 ns.
[8] The positron emission tomography device according to any one of [4] to [7], wherein a gamma-ray absorption coefficient of the scintillator is equal to or greater than 70%.
[9] The positron emission tomography device according to any one of [4] to [7], wherein a gamma-ray absorption coefficient of the scintillator is equal to or less than 50%.
[10] A signal processing method including generating image data, based on an electric signal group output from a radiation detector,
   wherein the signal processing method recognizes the electric signal group as a processing target, and
   the electric signal group includes at least part of an electric signal group meeting requirements described below:
   the electric signal group is an electric signal group with a signal value within a predetermined range, the electric signal group corresponding to a gamma ray with energy equal to or less than 375 keV;
   the predetermined range is equal to or greater than 50% and equal to or less than 80% relative to a 100% signal value; and
   the 100% signal value is a signal value detected when a gamma ray with energy of 511 keV enters a radiation detection element in a radiation detector and is totally absorbed by the radiation detection element.
[11] A signal processing method including generating image data, based on an electric signal group output from a radiation detector,
   wherein the signal processing method recognizes the electric signal group as a processing target, and
   the electric signal group includes at least part of an electric signal group corresponding to a gamma ray with an energy value within a predetermined range, and the predetermined range is equal to or greater than 232 keV and equal to or less than 340 keV.
[12] A positron emission tomography method including at least steps (a), (b), and (c) described below:
   (a) a scintillation step of converting radiation into an electromagnetic wave by using a scintillator receiving radiation and emitting an electromagnetic wave;
   (b) a conversion-output step of receiving an electromagnetic wave emitted from the scintillator, converting the received electromagnetic wave into a pulse-shaped electric signal, and outputting the resulting signal; and
   (c) a signal processing step including a step of performing signal processing by the signal processing method according to [10] or [11].
[13] The positron emission tomography method according to [12], wherein the scintillator meets a characteristic described below:
   intensity of inherent background of a scintillator is equal to or less than 200 Hz/cm³ in a range of a signal value being 10 to 120% with a signal value of the pulse-shaped electric signal when a gamma ray with energy of 511 keV enters the scintillator and is totally absorbed by the scintillator as 100%.
[14] The positron emission tomography method according to [12] or [13], wherein a time window in the conversion-output section is equal to or less than 180 ns.
[15] The positron emission tomography method according to any one of [12] to [14], wherein a fluorescence decay time (DT) of the scintillator when the scintillator is irradiated with a gamma ray is equal to or less than 25 ns.
[16] The positron emission tomography method according to any one of [12] to [15], wherein a gamma-ray absorption coefficient of the scintillator is equal to or greater than 70%.
[17] The positron emission tomography method according to any one of [12] to [15], wherein a gamma-ray absorption coefficient of the scintillator is equal to or less than 50%.

### EFFECTS OF THE INVENTION

The present invention can provide a signal processing system, a positron emission tomography device, and a positron emission tomography method that improve detection efficiency compared with conventional systems, devices, and methods. Further, the present invention can provide a signal processing system, a positron emission tomography device, and a positron emission tomography method that improve an S/N value by decreasing a total amount of radiation by improvement of detection efficiency and consequently decreasing random coincidences.

Further, by using a scintillator material with a small inherent background value, the present invention can provide a positron emission tomography device enabling a tremendously improved S/N value and further can provide a positron emission tomography device and a positron emission tomography method that enable downsizing by eliminating the need for radiation absorption in a scintillator and thereby reducing the thickness of the scintillator.

### BRIEF DESCRIPTION OF THE DRAWINGS

[FIG. 1] FIG. 1 is a cross-sectional view of a simulation space simulating a radiation imaging device according to an embodiment of the present invention in a Y-Z plane.
[FIG. 2] FIG. 2 is a cross-sectional view of the simulation space simulating the radiation imaging device according to the embodiment of the present invention in an X-Y plane.

### MODE FOR CARRYING OUT THE INVENTION

While embodiments of the present invention are described in detail below, the descriptions are examples (representative examples) of the embodiments of the present invention and do not limit the present invention to the content of the descriptions within the spirit and scope of the present invention.

A "signal value" herein is a parameter logarithmically representing a time-integrated value of a pulse-shaped electric signal as a relative numerical value and is so-called signal intensity.

A numerical value range represented by using "to" herein means a range including numerical values described before and after "to" as a lower limit and an upper limit, and "A to B" means equal to or greater than A and equal to or less than B.

Further, "a plurality of" herein means "equal to or greater than 2."

Further, while a plurality of embodiments will be described below, a condition in each embodiment may be applied to another embodiment within the scope of application.

### <Positron Emission Tomography Device>

The present invention according to an embodiment is a positron emission tomography device. The positron emission tomography device according to an embodiment of the present invention may be simply referred to as a "positron emission tomography device."

The positron emission tomography device includes a plurality of radiation detector sections each converting radiation into an electric signal, and a signal processing system generating image data, based on an electric signal group composed of the electric signals (converting the electric signal group into an image by signal processing).

### (Radiation Detector Section)

The radiation detector section in the positron emission tomography device receives radiation and outputs an electric signal.

While the configuration of the radiation detector section is not particularly limited, the section normally includes a radiation detection element receiving radiation and outputting an electric signal and is preferably in a form of an array including a plurality of the radiation detection elements from the viewpoint of acquiring an image, based on positional information of radiation. A radiation detection element herein refers to a minimum unit (such as a unit corresponding to one pixel) having a function of changing radiation into an electric signal.

A radiation detector section according to an embodiment at least includes a scintillator section including a scintillator receiving radiation and emitting an electromagnetic wave and a conversion-output section receiving an electromagnetic wave emitted from the scintillator, converting the received electromagnetic wave into a pulse-shaped electric signal, and outputting the resulting signal.

The radiation detection element according to the embodiment includes one scintillator (scintillator element) related to each radiation detection element and a conversion-output section related to the one scintillator (conversion-output element).

The radiation detector section according to another embodiment at least includes (a) one or more radiation detection elements each at least including a semiconductor member receiving radiation and generating an electron or a positive hole, and a pair of electrodes placed in such a way as to sandwich the semiconductor member in between, and (b) a transmission section transmitting an electron or a positive hole detected by the each radiation detection element to the signal processing system as an electric signal. By applying voltage between the two electrodes included in the radiation detection element, the electron or the positive hole generated by the semiconductor element reaches an electrode, and the radiation can be detected as an electric signal.

The radiation detection element according to the embodiment may be an element including a semiconductor member composed of a semiconductor material that may constitute a minimum unit related to radiation detection, similarly to one scintillator (scintillator element) according to the embodiment including the scintillator section, and an electrode related to the semiconductor member; and the element may further include a transmission section related to the semiconductor member.

The radiation detector section is desirably placed in such a way as to enclose a position where placement of a subject is assumed and may exist concentrically around the position of the subject or on a curved surface corresponding to a cylinder the radius of which is equidistant from the central axis of the subject, may be placed on a curved surface adjusted to the shape of the subject, may be able to move on the curved surfaces during imaging, or may be formed to be able to change placement according to the subject. While not being particularly limited, the distance between the surface of the subject and the radiation detection section during use is normally equal to or less than 50 cm, preferably equal to or less than 30 cm, and more preferably equal to or less than 10 cm; and while not being particularly limited, the lower limit is normally equal to or greater than 1 mm. Downsizing of the device is more enhanced as the distance to the subject becomes closer.

Further, the positron emission tomography device may be provided with an outer wall part made of a material with high radiation stopping power, such as lead, in order to prevent flying of background radiation from outside and penetration of radiation generated in the device to the outside of the device.

While the case of the radiation detector section including a scintillator section including a scintillator receiving radiation and emitting an electromagnetic wave, and a conversion-output section converting the electromagnetic wave into an electric signal will be described below as an example of the positron emission tomography device, the present embodiment is not limited thereto.

Further, placement of the device sections in the positron emission tomography device is not particularly limited and may be appropriately determined following a generally known positron emission tomography device.

### <Scintillator Section>

The scintillator section in the positron emission tomography device (may also be hereinafter simply described as the "scintillator section") is not particularly limited as long as the section includes a scintillator, receives radiation being a measurement target, and emits an electromagnetic wave. The scintillator section may include a plurality of scintillators. Further, the scintillator section may include a laminate of the same type or different types of scintillators and can acquire preferable positional resolution even with a laminate of different types. Furthermore, the scintillator section may include a reflection layer exhibiting reflectivity against an electromagnetic wave emitted, by the scintillator, between the different types of scintillators and/or on a radiation incident plane of the scintillator.

### <Scintillator>

The scintillator included in the scintillator section (may also be hereinafter simply described as the "scintillator") is excited by receiving energy and emits an electron or light. When light is emitted, the light is preferably emitted in a wavelength region equal to or greater than 160 nm and equal to or less than 700 nm from the viewpoint of enhancing conversion efficiency from an electromagnetic wave to an electric signal in the conversion-output section. Further, the scintillator has an emission peak wavelength in a wavelength region preferably equal to or greater than 300 nm and more preferably equal to or greater than 350 nm, and preferably equal to or less than 600 nm and more preferably equal to or less than 550 nm. Examples of energy related to the excitation include an electromagnetic wave, an electron beam, and ionizing radiation. Examples of the ionizing radiation include an X-ray, a γ-ray, a β-ray, an α-ray, and a neutron beam, and a γ-ray is preferable.

A radiation incident plane of each scintillator element is not particularly limited and may be, for example, planar or curved; and the area of the radiation incident plane of the element is not particularly limited but is normally equal to or less than 36 mm², preferably equal to or less than 9 mm², and more preferably equal to or less than 4 mm². A smaller radiation incident plane of the scintillator element can further improve spatial resolution.

Since a scintillator has been conventionally required to absorb entire radiation energy, the thickness of the scintillator has been required to be set large; however, the scintillator according to the present embodiment has only to have a thickness allowing absorption of at least {1 - (1/e)} of the entire radiation energy. The thickness of the scintillator refers to the length of the scintillator with respect to a radiation incident direction.

Specifically, while the thickness of the scintillator allowing absorption of at least {1 - (1/e)} of the entire radiation energy is appropriately determined based on the energy and the type of the radiation, and the density and the effective atomic number of the scintillator, the thickness is normally equal to or greater than 1 mm, preferably equal to or greater than 5 mm, more preferably equal to or greater than 10 mm, and yet more preferably equal to or greater than 20 mm and is normally equal to or less than 100 mm, preferably equal to or less than 60 mm, more preferably equal to or less than 50 mm, and yet more preferably equal to or less than 40 mm. More specifically, for example, when the radiation is a gamma ray with energy of 511 keV and the density and the effective atomic number of the material of the scintillator are equal to or close to those of LSO, that is, the material has a density of 7 to 8 g/cm³ and an effective atomic number of 60 to 70, the thickness of the scintillator is normally equal to or greater than 1 mm, preferably equal to or greater than 2 mm, more preferably equal to or greater than 5 mm, yet more preferably equal to or greater than 10 mm, and particularly preferably equal to or greater than 15 mm and is normally equal to or less than 60 mm, preferably equal to or less than 50 mm, more preferably equal to or less than 40 mm, and more preferably equal to or less than 30 mm. The thickness being equal to or greater than the lower limit of the range enables securement of absorption efficiency of radiation and acquisition of preferable radiation detection efficiency. Further, the thickness being equal to or less than the upper limit of the range enables acquisition of preferable positional resolution. Note that since the aforementioned range can be appropriately changed and applied based on the energy and the type of the radiation, and the density and the effective atomic number of the scintillator as described above, the present embodiment is not limited to the aforementioned range.

The scintillator itself may emit radiation. The radiation is herein referred to as inherent background radiation. Examples of the inherent background radiation include an α-ray, a β-ray, a γ-ray, and an X-ray. Further, for example, indirectly generated radiation such as a case of the scintillator emitting a positron and a γ-ray originating from the positron is herein also handled as inherent background radiation.

Inherent background intensity may be represented by generation frequency (Hz/cm³) of radiation generated per 1 cm³ of the scintillator and per second in an energy section.

The intensity of the inherent background radiation is normally equal to or less than 350 Hz/cm³, preferably 250 Hz/cm³, more preferably equal to or less than 200 Hz/cm³, yet more preferably equal to or less than 150 Hz/cm³, and particularly preferably equal to or less than 100 Hz/cm³ in an energy band of 0.1 to 1.2 times as much as initial energy of radiation being an observation target of the positron emission tomography device. While the lower limit is not particularly limited, and a smaller value is more preferable, the value is normally equal to or greater than 0 Hz/cm³ and may be equal to or greater than 10 Hz/cm³.

For example, when a gamma ray originating from a positron emission nuclide is used, the initial energy of radiation being an observation target of the positron emission tomography device is 511 keV, and therefore the energy band equal to or greater than 0.1 times and equal to or less than 1.2 times as much as the initial energy becomes equal to or greater than 51.1 keV and equal to or less than 612.1 keV.

The intensity of the inherent background radiation may also be observed in a form of a pulse-shaped electric signal being converted from the inherent background radiation and being output by the conversion-output section; and when being observed by the method, the intensity of the inherent background radiation is normally equal to or less than 350 Hz/cm³, preferably equal to or less than 250 Hz/cm³, more preferably equal to or less than 200 Hz/cm³, yet more preferably equal to or less than 150 Hz/cm³, especially preferably equal to or less than 100 Hz/cm³, particularly preferably equal to or less than 50 Hz/cm³, yet particularly preferably equal to or less than 10 Hz/cm³, and most preferably equal to or less than 5 Hz/cm³ in a range of 10 to 120% of the signal value with a signal value of the pulse-shaped electric signal generated when a gamma ray with energy of 511 keV enters the scintillator and is totally absorbed by the scintillator as 100%. While the lower limit is not particularly limited and a smaller value is more preferable, the value is normally equal to or greater than 0 Hz/cm³ and may be equal to or greater than 1 Hz/cm³.

A smaller amount of inherent background radiation reduces mixing of noise, thereby eliminating confusion between a low-energy scattered coincidence and noise, or eliminates the need for setting a high range of a signal used as the signal for noise elimination and therefore enables efficient use of scattered coincidences, thereby enabling increase in the number of events relative to the total amount of radiation.

By performing measurement in a hermetically sealed container made of a material with high radiation stopping power, measurement of the inherent background intensity can be performed by adding up the number of coincident events of light emitted by radiation from the scintillator itself with as much elimination of environmental radiation such as a cosmic ray as possible. By previously performing calibration of an amount of emitted light and energy of radiation, energy of radiation can be calculated from an amount of emitted light at measurement, and inherent background intensity for each value of energy of radiation can be determined. For example, a container including a layer made of lead with a thickness of around 10 cm and a layer made of oxygen-free copper with a thickness of around 1 cm inside the layer may be used as the hermetically sealed container.

### <Fluorescence Decay Time of Scintillator>

The fluorescence decay time (DT) of a scintillator when the scintillator is irradiated with a gamma ray is normally equal to or less than 50 ns, preferably equal to or less than 35 ns, more preferably equal to or less than 25 ns, yet more preferably equal to or less than 20 ns, particularly preferably equal to or less than 15 ns, and most preferably equal to or less than 12 ns. The lower limit is not particularly limited but is normally equal to or greater than 0.1 ns.

As the DT becomes shorter, time resolution of a signal is improved and a time window to be described later can be set shorter; and therefore the S/N value can be improved by eliminating a random coincidence.

The DT of the scintillator can be determined by the following method. Specifically, the intensity of an electromagnetic wave emitted from the scintillator attenuates exponentially over time, and a signal value of an output electric signal converted from the electromagnetic wave by the conversion-output section consequently attenuates exponentially. Therefore, the DT can be found by making a plot with signal values and time as axes and performing fitting on a curve composed of signal values for respective times by using an exponential function.

A shorter DT of the scintillator enables a shortened time window; and when the inherent background intensity is sufficiently low, each of time intervals at which inherent background radiation is generated becomes sufficiently long relative to the time window, and therefore background radiation can be excluded from detection targets in simultaneous measurement. In other words, existence of inherent background can be neglected, and therefore when radiation with energy less than 511 keV but with a certain energy value or greater excluding relatively low-energy radiation such as back scattering is detected, the radiation can be calculated as a true coincidence. Specifically, the DT is preferably equal to or less than 50 ns and the inherent background intensity is preferably equal to or less than 250 Hz/cm³, and more preferable ranges of the DT and the background intensity are as described above, respectively.

While the fluorescence intensity after an elapse of 100 ns from a time when the scintillator is irradiated with a gamma ray and the fluorescence intensity becomes a maximum value is not particularly limited, the intensity is normally equal to or less than 5%, preferably equal to or less than 4%, more preferably equal to or less than 3%, yet more preferably equal to or less than 2%, and particularly equal to or less than 1.5% with the maximum value of the fluorescence intensity as 100%. The lower limit is not particularly limited and is normally equal to or greater than 0% or equal to or greater than 0.001%. Thus, a scintillator material contributing to radiographic inspection with high time resolution can be provided by very fast fluorescence attenuation, and sufficiently low fluorescence intensity after an elapse of a predetermined time.

### <Energy Absorption Coefficient for Radiation>

In order to detect radiation, the scintillator preferably absorb part or all of energy when the radiation enters. Assuming the ratio of energy absorbed by the scintillator to energy of incident radiation to be an energy absorption coefficient, high radiation detection efficiency can be acquired due to a high energy absorption coefficient of the scintillator, according to the embodiment of the present invention, similarly to a conventional positron emission tomography device. From such a viewpoint, the energy absorption coefficient of the scintillator, the gamma-ray absorption coefficient in particular, is preferably equal to or greater than 50%, preferably equal to or greater than 70%, more preferably equal to or greater than 80%, and yet more preferably equal to or greater than 90%; and while not being particularly limited, the upper limit may be 100%.

Further, according to another embodiment, the radiation imaging device can be downsized by reducing the thickness of the scintillator due to a low energy absorption coefficient of the scintillator, contrary to the above. From such a viewpoint, the energy absorption coefficient of the scintillator, the gamma-ray absorption coefficient in particular, is normally equal to or less than 90%, preferably equal to or less than 70%, more preferably equal to or less than 50%, and yet more preferably equal to or less than 30%; and the lower limit is normally equal to or greater than 10%. Note that when the energy absorption coefficient is not 100% in a conventional positron emission tomography device, scattered radiation generated in the scintillator becomes noise and the S/N value is reduced; however, according to the embodiment of the present invention, scattered radiation generated in the scintillator is negligible due to the material having a short fluorescence lifetime while having a certain degree of radiation detection efficiency and inherent background of the material being negligible, and therefore a high S/N value can be secured even when the energy absorption coefficient is low, as described above.

An amount of emitted light of the scintillator is normally equal to or greater than 1000 Ph/MeV, preferably equal to or greater than 5000 Ph/MeV, and more preferably equal to or greater than 20000 Ph/MeV. While not being particularly limited, a higher upper limit further improves radiation detection sensitivity.

As the density of the scintillator increases and the effective atomic number increases, the energy absorption coefficient of radiation increases, and the detection efficiency of the radiation improves. From this viewpoint, the density of the scintillator is normally equal to or greater than 4 g/cm³, preferably equal to or greater than 6 g/cm³, yet more preferably equal to or greater than 7 g/cm³, particularly preferably equal to or greater than 7.5 g/cm³, and yet particularly preferably equal to or greater than 8 g/cm³; and while not being particularly limited, the upper limit is normally equal to or less than 12 g/cm³.

Further, the effective atomic number of the scintillator is normally equal to or greater than 30, preferably equal to or greater than 40, more preferably equal to or greater than 45, yet more preferably equal to or greater than 50, particularly preferably equal to or greater than 55, and especially preferably equal to or greater than 60; and while not being particularly limited, the upper limit may be, for example, equal to or less than 100.

Note that the effective atomic number may be determined with reference to the description in Medical Physics, 39 (2012), p. 1769, based on the composition of the scintillator.

While the type of scintillator is not particularly limited as long as various characteristics required of the aforementioned scintillator are included and the essence of the present invention is not lost, for example, when radiation is a gamma ray, BGO, a plastic scintillator, an organic scintillator, lutetium orthosilicate (LSO) or a yttrium or gadolinium substitution product scintillator of the LSO (LYSO or LGSO), a hafnium-oxide-based scintillator (such as BaHfO₃, SrHfO₃, or CaHfO₃), LuBr₃, Nd-doped LaF₃, Yb-doped garnet (YAG:Yb, or YbAG), or the like may be used.

Among the aforementioned types of scintillators, each of the hafnium-oxide-based scintillator, the plastic scintillator, BGO, Nd-doped LaF₃, and Yb-doped garnet (YAG:Yb or YAG) has a preferable background intensity equal to or less than 200 Hz/cm³ from the viewpoint of low intensity of the inherent background; and Nd-doped LaF₃ (DT = 20 ns or less), Yb-doped garnet (DT = 50 ns or less), and the hafnium-oxide-based scintillator (DT = 10 to 20 ns) are particularly preferable from the viewpoint of a short DT. Note that Yb-doped garnet with a density 4.56 g/cm³ and an effective atomic number 32.6, and the hafnium-oxide-based scintillator with a density 8.1 g/cm³ and an effective atomic number 64 are also preferable from the viewpoint of density and an effective atomic number. Only one type out of the scintillators may be used or two or more types may be used in any combination. Further, part of the composition of a scintillator may be converted by another element.

The aforementioned scintillators can be manufactured by generally known methods, and a commercially available product may also be used.

### <Conversion-output Section>

The conversion-output section in the positron emission tomography device according to the present embodiment (may also be hereinafter simply described as the "conversion-output section") is not particularly limited as long as the section can receive an electromagnetic wave emitted by the scintillator and output a related electric signal; and a generally known product may be used.

The conversion-output section preferably outputs an electric signal with a signal value related to energy of the electromagnetic wave, and more preferably, the signal value is proportional to the energy. In this case, the positron emission tomography device can identify energy information of radiation according to the signal value and distinguish between background radiation, scattered radiation, a scattered coincidence, a true coincidence, and the like. Further, the conversion-output section may include a mechanism for amplifying a received signal value for improved sensitivity.

The conversion-output section may include a light receiving section (may be a photodetector) receiving an electromagnetic wave emitted from the scintillator and a signal output section outputting an electromagnetic wave received by the light receiving section as an electric signal. The light receiving section and the signal output section may be included as separate members or may be included as an integrated member. The form of the light receiving section is not particularly limited; and a generally known form may be used, and a commercially available product may be used. Further, the form of the signal output section is not particularly limited, and for example, a circuit accumulating electric charge and a circuit outputting electric charge as an electric signal at a certain timing or according to an accumulated amount of electric charge may be appropriately used in combination; and a generally known form may be used, and a commercially available product may be used.

The type of the conversion-output section is not particularly limited; and a generally known type may be used, and a commercially available product may be used. Further, the time resolution of the conversion-output section preferably allows distinction between signals at time intervals substantially the same as the DT of the scintillator or shorter. For example, a photomultiplier tube, a Si avalanche photodiode, and a Si Geiger-mode avalanche photodiode may be used; and out of the devices, a multianode-type or array-type position sensitive conversion-output section is more preferable from the viewpoint of preventing decrease in a detectable area due to a gap generated between conversion-output sections.

### <Time Window>

In the coincidence counting method, a time window in the conversion-output section is denoted by τ in units of nanoseconds (ns). Denoting the measurement starting time by t = 0 and when two different detectors measure radiation in a time period (n-1)τ ≤ t ≤ nτ (where n is a natural number) for times t = τ, 2τ, 3τ, ..., one coincidence is assumed to have occurred (one event is assumed to have occurred). For example, the time window may be equal to or less than 200 ns and is preferably equal to or less than 180 ns, more preferably equal to or less than 160 ns, yet more preferably equal to or less than 140 ns, and particularly preferably equal to or less than 120 ns; and while not being particularly limited, the lower limit is normally equal to or greater than 10 ns. By setting the time window short, random coincidences can be decreased and the S/N value can be improved by eliminating noise; and furthermore, the cycle time of measurement can be shortened.

### transmission Section>

The positron emission tomography device according to the present embodiment may be provided with a transmission section transmitting an electron or a positive hole generated by the semiconductor member to the signal processing system as an electric signal, as described above. The transmission section is also referred to as an electric signal output section in this field and may have a generally known form; and a commercially available product may be used. Examples of the form include a form including an electric charge accumulation circuit such as a capacitor. Furthermore, the transmission section may include an amplifier circuit (such as an amplifier or an integrating amplifier circuit) amplifying information of electric charge or an electric signal, or a glitch elimination circuit such as a sample-and-hold circuit, the ground connectable to a circuit that can accumulate an electric charge and a switch switching ON/OFF connection between the ground and the circuit, and a filter circuit (such as a low-pass filter or a high-pass filter) eliminating unnecessary low- and highfrequency noise. Appropriate inclusion of the aforementioned circuits enables sensitivity improvement, noise elimination, elimination of residual electric charge after electric signal output, or the like, thereby improving precision of the electric signal.

### <Structure of Radiation Detector Section and Surrounding Part>

The radiation detector section may be used in combination with a separate member that can detect or shield radiation by integrating the separate member or placing the separate member in a surrounding part.

The separate member is not particularly limited as long as the member is a member or equipment that can detect or shield radiation, and for example, a radiation conversion member or a radiation shielding member may be used; and, for example, a semiconductor converting radiation into an electric signal or a common scintillation material converting radiation into an electromagnetic wave similarly to the scintillator according to the embodiment may be used as the radiation conversion member, and such a member may be used as, for example, an active collimator. While a collimator transmitting only a specific direction using lead, tungsten or the like, a coded collimator, or the like may be used as the radiation shielding member, another material or shape shielding radiation may also be used. The separate member may be placed between a subject and the scintillator section (a previous stage of the scintillator) or may be placed on the opposite side of the subject viewed from the scintillator section (a subsequent stage of the scintillator). Such placement allows use as a signal selection member or a trigger.

### <Signal Processing System>

The present invention according to an embodiment is a signal processing system. The signal processing system according to the present embodiment may also be hereinafter simply described as the "signal processing system." The signal processing system may be used in a common positron emission tomography device or may be used as a component in the positron emission tomography device according to the aforementioned embodiment of the present invention.

The signal processing system generates image data, based on an electric signal output from a radiation detector. Specifically, when two radiation detection elements detect signals with signal values equal to or greater than a threshold value in a certain time span (time window), data collection of the signal values of electric signals is started; and an energy value is calculated from the signal values of the electric signals, and the radiation source location (limited to one dimension) is calculated from the positions of the two radiation detection elements receiving the radiation. At that time, detected time information may be acquired. Otherwise, time information may also be acquired when a signal is detected only by one radiation detection element, and simultaneousness may be determined by comparison with time information of another radiation detection element. Then, the position (point) is determined by accumulating statistics of the calculated radiation source locations.

Furthermore, the likelihood of a detected position may be calculated for the positional information determined by the aforementioned method, based on the positional resolution of the detector, or the like. Finally, by reflecting the aforementioned position and the likelihood thereof, a final image can be reconstructed.

A known method may be used as the method of the reconstruction, and for example, an ordered subset expectation maximization method (OSEM method) or the like may be used.

Furthermore, in the signal processing system, image reconstruction may be performed by performing weighting on the likelihood of the position, based on energy information, for an electric signal originating from radiation with energy with a value equal to or less than the energy value intrinsically possessed by radiation, such as equal to or less than 511 keV in a case of a gamma ray originating from a positron emission nuclide.

For example, as a method for evaluating the likelihood of a position for a scattered coincidence, based on the energy information, a degree of angle change due to Compton scattering can be predicted based on the energy information, and based on the prediction, a region where a radiation source position may exist can be predicted.

Without being particularly limited, the form of image data generated by the signal processing system may be, for example, an image acquired by, after estimating the source position of a positron from which radiation originates for each event detected as simultaneous measurement, plotting the concentration density of the positron source (tracer) for each position.

Employment of the aforementioned technique enables an improved S/N value.

In the positron emission tomography method in medical care, by taking advantage of a characteristic that sugar such as glucose and the like tend to be localized in a cancerous cell, a radiation source position is determined to be a position where a cancerous cell may exist, by using a positron emission nuclide such as fluorodeoxyglucose acquired by substitution of ¹⁸F, being a radioisotope of fluorine as a tracer. Examples of a positron emission nuclide include ¹⁵O, ¹¹C, and ¹³N as respective radioisotopes of oxygen, carbon, and nitrogen but are not limited thereto. Further, examples of a tracer to which a positron emission nuclide is attached include water and acetic acid in addition to sugar glucose.

Note that a tracer is input to a target object or a patient by injection, inhalation, or the like. While radiation to be input is normally around 1 MBq to around 1 GBq, secure acquisition of a video image with a lower dose is required since an effect on a patient can be held down by reducing a dose.

A signal processing system according to an embodiment is a signal processing system generating image data, based on an electric signal output from a radiation detector, and the signal system recognizes the electric signal group as a processing target; and
the electric signal group includes at least part of an electric signal group meeting the following requirements:
the electric signal group is an electric signal group with a signal value within a predetermined range, the electric signal group corresponding to a gamma ray with energy equal to or less than 375 keV;
the predetermined range is equal to or greater than 50% and equal to or less than 80% relative to a 100% signal value; and
the 100% signal value is a signal value detected when a gamma ray with energy of 511 keV enters a radiation detection element in the radiation detector and is totally absorbed by the radiation detection element. The signal processing system recognizing such an electric signal group as a processing target can improve detection efficiency, further decrease a total amount of radiation by the improvement of detection efficiency, and consequently improve an S/N value by decreasing random coincidences, compared with conventional systems.

The predetermined range related to the signal value is normally equal to or greater than 30%, preferably equal to or greater than 40%, more preferably equal to or greater than 50%, yet more preferably equal to or greater than 52.5%, and yet especially preferably equal to or greater than 55% and is normally equal to or less than 80%, preferably equal to or less than 70%, more preferably equal to or less than 65%, and yet more preferably equal to or less than 60% relative to the aforementioned 100% signal value. By the predetermined range being equal to or greater than the aforementioned lower limit, an electric signal generated by low-energy radiation such as back scattering radiation generated by scattering of radiation on an outer wall part of the device can be suitably excluded as noise, and by the predetermined range being equal to or less than the aforementioned upper limit, more electric signals including electric signals originating from scattered coincidences can be employed.

From another viewpoint, a signal processing system according to an embodiment is a signal processing system generating image data, based on an electric signal group output from a radiation detector; and the signal processing system recognizes the electric signal group as a processing target,
the electric signal group includes at least part of an electric signal group corresponding to a gamma ray with an energy value within a predetermined range, and the predetermined range is equal to or greater than 232 keV and equal to or less than 340 keV. The signal processing system recognizing such an electric signal group as a processing target can improve detection efficiency, further decrease a total amount of radiation by the improvement of detection efficiency, and consequently improve an S/N value by decreasing random coincidences, compared with conventional systems.

Note that an electric signal "corresponding to" a gamma ray with certain energy herein means an electric signal generated when a gamma ray with the certain energy value enters a radiation detection element and is absorbed.

While not being particularly limited, the predetermined range related to energy of the gamma ray is normally equal to or greater than 180 keV, preferably equal to or greater than 200 keV, more preferably equal to or greater than 220 keV, yet more preferably equal to or greater than 232 keV, and yet especially preferably equal to or greater than 250 keV and is normally equal to or less than 420 keV, preferably equal to or less than 375 keV, more preferably equal to or less than 340 keV, yet more preferably equal to or less than 320 keV, yet especially preferably equal to or less than 300 keV, and particularly preferably equal to or less than 280 keV. By the predetermined range related to the energy value of the gamma ray being equal to or greater than the aforementioned lower limit, an electric signal generated by low-energy radiation such as back scattering radiation generated by scattering of radiation on an outer wall part of the device can be suitably excluded as noise, and by the predetermined range related to the energy value of the gamma ray being equal to or less than the aforementioned upper limit, more electric signals including electric signals originating from scattered coincidences can be employed.

A signal processing system "including" a certain electric signal as a processing target means assuming the electric signal as a true coincidence and using source position information, energy information, and the like of a gamma ray estimated from the electric signal for the purpose of image data generation. Further, according to the embodiment, a processing target has only to use at least part of electric signals meeting a condition related to the aforementioned "predetermined range," or all electric signals meeting the condition may be set as a processing target.

A signal processing system according to an embodiment recognizes every electric signal with a signal value equal to or greater than a certain threshold value as at least a processing target out of electric signals output from a radiation detector. The threshold value is normally equal to or less than 50%, preferably equal to or less than 40%, more preferably equal to or less than 30%, and yet more preferably equal to or less than 20% and is normally equal to or greater than 5%, preferably equal to or greater than 10%, and more preferably equal to or greater than 15% relative to a signal value when a gamma ray with energy of 511 keV enters a scintillator and is totally absorbed by the scintillator (with the signal value as 100%). Note that the signal processing system may recognize an electric signal with a signal value equal to or less than the threshold value as a processing target within a range in which effects of the present invention are provided, such as a range in which noise generated by low energy such as back scattering radiation described below, or the like is not included.

By the threshold value being equal to or greater than the lower limit of the range, an electric signal generated by low-energy radiation such as back scattering radiation generated by scattering of radiation on an outer wall part of the device can be suitably excluded as noise, and by the threshold value being equal to or less than the upper limit of the range, more electric signals including electric signals originating from scattered coincidences can be employed.

While only a gamma ray at 511 keV is calculated as a true coincidence in conventional PET methods, an event detected at 511 keV or less can also be calculated as a true coincidence when the positron emission tomography device according to the present embodiment is used.

Note that a gamma ray with energy equal to or greater than 232 keV is normally recognized as a processing target in order to exclude back scattering radiation from processing targets.

### <Other Device Sections>

The positron emission tomography device may be provided with a device section other than the aforementioned device section, and for example, a computer tomography device or a cooling device may be provided.

A method for manufacturing the positron emission tomography device is not particularly limited, and the device may be manufactured following a generally known method in such a way that each of the aforementioned device sections is placed at a desired position.

### (Signal Processing Method)

The present invention according to an embodiment is a signal processing method including generating image data, based on an electric signal group output from a radiation detector; and
the signal processing method recognizes the electric signal group as a processing target,
the electric signal group includes at least part of an electric signal group meeting the following requirements in a signal processing system:
   the electric signal group is an electric signal group with a signal value within a predetermined range, the electric signal group corresponding to a gamma ray with energy equal to or less than 375 keV;
the predetermined range is equal to or greater than 50% and equal to or less than 80% relative to a 100% signal value; and
the 100% signal value is a signal value detected when a gamma ray with energy of 511 keV enters a radiation detection element in the radiation detector and is totally absorbed by the radiation detection element. The signal processing method recognizing such an electric signal group as a processing target can improve detection efficiency, further decrease a total amount of radiation by the improvement of detection efficiency, and consequently improve an S/N value by decreasing random coincidences, compared with conventional methods.

Further, the present invention according to an embodiment is a signal processing method including generating image data, based on an electric signal group output from a radiation detector, the method being used in radiation imaging; and
the signal processing method recognizes the electric signal group as a processing target, and
the electric signal group includes at least part of an electric signal group corresponding to a gamma ray with an energy value within a predetermined range, and the predetermined range is equal to or greater than 232 keV and equal to or less than 340 keV. The signal processing method recognizing such an electric signal group as a processing target can improve detection efficiency, further decrease a total amount of radiation by the improvement of detection efficiency, and consequently improve an S/N value by decreasing random coincidences, compared with conventional methods.

For example, the signal processing method can be provided by using the signal processing system. All conditions and features in the signal processing method, such as equipment, a device, a processing condition, and a preferable range of a signal value or gamma-ray energy value that are to be used and effects to be provided are not limited as long as the objects of the invention are achieved and may be applied by reading "signal processing system" as "signal processing method" and appropriately reading expressions such as "be provided" and "include" as "use" in the description about the aforementioned signal processing system.

Further, the signal processing method can be used in (c) signal processing step in the following positron emission tomography method.

### (Positron Emission Tomography Method)

The present invention according to an embodiment is a positron emission tomography method including at least the following steps (a), (b), and (c):
(a) a scintillation step of converting radiation into an electromagnetic wave by using a scintillator receiving radiation and emitting an electromagnetic wave;
(b) a conversion-output step of receiving an electromagnetic wave emitted from the scintillator, converting the received electromagnetic wave into a pulse-shaped electric signal, and outputting the resulting signal; and
(c) a signal processing step including a step of performing signal processing by the signal processing method.

For example, each of the steps (a), (b), and (c) can be provided by using the positron emission tomography device. All conditions and features in each of the steps (a), (b), and (c), such as the composition, the structure, and the characteristic of a device or a member to be used, a conversion-output condition, a signal processing condition, and effects to be provided by each of the conditions, are not particularly limited as long as the objects of the invention are achieved and may be applied by, for example, reading "positron emission tomography device," "scintillator section," "conversion-output section," and "signal processing system" as "positron emission tomography method," "scintillation step," "conversion-output step," and "signal processing step," respectively, and appropriately reading "be provided" and "include" as "use" in the description about the positron emission tomography device.

Further, any process other than the aforementioned image conversion process may be provided in the positron emission tomography method.

Further, a process of injecting a substance (tracer) acquired by substituting a positron emission nuclide (radiation source) for part of elements in a molecule localized in a cancerous cell into a patient being an imaging target and a process of, in a case of the positron emission tomography device including a columnar space part and a scintillator section being placed on a wall forming the columnar shape, guiding the patient to the space part may be provided in the positron emission tomography method.

The type of tracer described in the aforementioned positron emission tomography device may be similarly applied to the type of the aforementioned tracer.

### <Simulation Condition>

The effects of the present invention can be verified by simulation. A simulation can simulate a situation in which a positron imaging device and a subject are generated in a virtual space, and by generating a predetermined number of tracers emitting a predetermined type of radiation in a specific region in the subject, radiations the number of which is based on the number of tracers are emitted from the tracers and enter a scintillator in the positron imaging device. For example, a positron emission tomography device can be generated in a virtual space by using a program code for calculating an interaction between a substance, and a particle or a photon, the program using the Monte Carlo method.

An alpha ray, a beta ray, a gamma ray, an X-ray, or the like may be set as radiation, and a difference between results based on energy information can be predicted by setting initial energy of the radiation. Only one type of radiation may be set, or two or more types may be set.

### <Calculation Program>

While an existing method may be used as the method for calculating an interaction between a substance, and a particle or a photon without being particularly limited, for example, the Monte Carlo method may be used. By the method, for example, a situation in which emitted radiation originating from a tracer is scattered, diffracted, or absorbed in a subject, in a space between the subject and the scintillator section, inside the scintillator, or on the outer wall, thereby changing energy or an angle of the radiation can be calculated.

Further, by setting a molar ratio, density, a shape, a region, or the like of an element in each substance constituting the subject, the space, the scintillator section, the outer wall, or the like, behavior of radiation entering each of the actual substances can be predicted, and a result based on the type, placement, the thickness, the shape, or the like of the scintillator can be predicted.

### <Radiation Detection Section and Background Radiation>

As for the radiation detection section, a situation in which only a scintillator section such as a scintillator block (scintillator unit) with a defined size is placed for simplification, an interaction occurs between radiation and the scintillator section when the radiation enters the scintillator unit in terms of calculation, and the radiation is detected with a predetermined probability can be calculated. When a plurality of scintillator units are placed, the scintillator units are preferably spaced at equal to or greater than 0.5 mm intervals.

As for background radiation, a result based on background radiation and a processing method thereof can be predicted by setting inherent background intensity of the scintillator and background generated from each part. While any energy distribution of background radiation can be used, a literature value may be applied when a known material is assumed.

Furthermore, energy resolution and a range of a signal value being a processing target may be set in the signal processing system, and an effect due to a change in the setting can also be predicted.

Inherent background radiation refers to a gamma ray, a beta ray, or the like being generated by the scintillator itself and not being caused by input of energy such as radiation from the outside of the scintillator, or a gamma ray or the like immediately generated inside the scintillator by pair annihilation of a positron emitted inside the scintillator. Since the intensity of such radiation has significant intensity according to the type of scintillator, the value of the intensity may be set for each scintillator and be reflected in the simulation.

While background radiation further includes continuous components being generated by Compton components in the beta ray and the gamma ray and not having a peak at specific energy in addition to the inherent background, the probability of occurrence (frequency) of the continuous components is normally equal to or less than 0.5 Hz/cm³, which is very low, and therefore may be neglected in this simulation.

Further, while a component originating from environmental background generated by an environment outside the device and originating from a cosmic ray or the like may exist as background, the probability of occurrence (frequency) of the component is normally equal to or less than 0.1 Hz/cm³, which is very low, and therefore may be neglected in this simulation.

### <Detection Technique, Detection Efficiency, and Calculation of S/N Value>

By classifying all radiations detected by the scintillator unit into radiations corresponding to electric signals to be recognized as processing targets in the signal processing system, that is, signals, and the remainder, that is, noises, based on energy information of each radiation, a value acquired by dividing the number of signals (S) by the number of noises (N) can be calculated as an S/N value. A criterion for distinguishing between the number of signals and the number of noises may be appropriately determined according to the detection method; and a conventional detection method may employ a criterion described in Comparative Example 1 included in Examples to be described later, and a detection method according to the present embodiment may employ a criterion described in Example 1 included in Examples to be described later. Note that, for example, a technique of limiting generated radiation to only one direction for convenience of calculation, finding detection efficiency by a single measurement technique, and then correcting the result to detection efficiency and an S/N value based on a simultaneous measurement technique may be employed in a simulation.

### <Energy Window and Energy Resolution>

In a simulation, only an event in a specific energy band (may also be hereinafter described as an energy window) may be employed as a signal (S) or a noise (N) related to single measurement for the purpose of eliminating an event detected in an energy band of back scattering or the like, and detection efficiency and an S/N value can be found based on the above. Further, an event with energy outside the energy window may be determined to be an event not employed in the aforementioned S/N value calculation.

For example, the energy window may be set in a range of several times as wide as an energy resolution (FWHM value), such as twice to six times, preferably three to five times, and more preferably four times around an initial value of energy possessed by detection target radiation (511 keV in the case of the PET device). While any energy resolution (FWHM value) can be employed, for example, the energy resolution may be set to around 5 to 20% relative to energy possessed by detection target radiation at generation (such as 511 keV) and may also be set to, for example, 10% or 15%.

### Examples

While the embodiment of the present invention will be described in more detail below by Examples, the present invention is not limited to Examples. First, simulation items with the same setting across Examples and Comparative Examples will be described.

### <Program and Coordinates Design>

A positron emission tomography device under the following condition was generated on a virtual space by using GEANT4 being a program code for calculating an interaction between a substance, and a particle or a photon by using the Monte Carlo method. Note that a position on the virtual space is a three-dimensional space composed of three axes being x-, y- and z-axes orthogonal to each other, and the center of a subject is set to the origin, that is, (x, y, z) = (0, 0, 0). Further, a direction in which a patient is inserted into the positron emission tomography device is set to the z-axis, and the positron emission tomography device is assumed to be concentrically placed relative to the z-axis. The positron emission tomography device includes a signal processing system that can perform a signal processing method described in each Example or Comparative Example to be described later.

### <Simulation Condition>

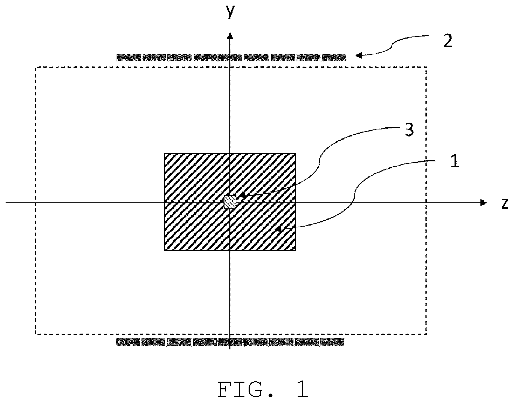

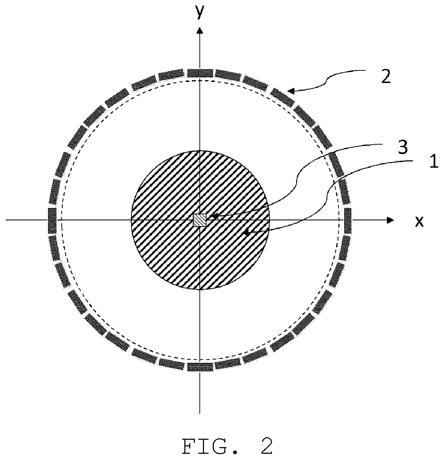

FIG. 1 and FIG. 2 illustrate placement of a subject 1, a cancerous part 3, and a scintillator section 2 (a scintillator unit or a scintillator in this case) in a simulation space. The subject was assumed to be a biologically equivalent substance (brain), and contents thereof were assumed to be hydrogen 64.44%, carbon 7.33%, nitrogen 0.95%, oxygen 27.01%, sodium 0.05%, phosphorus 0.08%, sulfur 0.04%, chlorine 0.05%, and potassium 0.05% in terms of a molar ratio. Further, the position of the subject was assumed to be centered on the origin as described above; and the size thereof was assumed to be a cylinder with a radius 8 cm and a height 10 cm (5 cm in each of the z-direction and the -z-direction) assuming the z-axis direction to be the height direction, and the density was assumed to be 1.03 g/cm³.

Next, a cancerous part was assumed to exist at the origin. The size of the cancerous part was assumed to be 1 cm, 1 cm, and 1 cm in the x-, y-, and z-directions, respectively. In other words, the position of the cancerous part was assumed to be in a range enclosed by (x, y, z) = (-0.5 cm, -0.5 cm, -0.5 cm) and (x, y, z) = (0.5 cm, 0.5 cm, 0.5 cm). A tracer was assumed to be evenly spread over the affected part, and a gamma ray at 511 keV was assumed to be emitted from the cancerous part range with a uniform probability.

The gamma rays were set to be generated at 100,000,000 per second. Note that the generation frequency of the gamma rays corresponds to around 100 MBq of radioactivity of the tracer.

### <Placement and Setting of Scintillator Section>

For simplification, a scintillator unit was placed in place of a radiation detector section in a simulation, and radiation and energy information were assumed to be detected by radiation entering the unit and interacting with a scintillator.

Schematic diagrams of the placement of the subject and the scintillator viewed from the x-axis direction and the y-axis direction are illustrated in FIG. 1 and FIG. 2, respectively.

First, 32 scintillator units were placed in a circularly symmetric manner at equal intervals on a cylindrical surface with a radius of 30.4 cm around the z-axis on the x-y plane in such a way that an interval between units in an inner circle part is 0.5 mm. Next, the same set of the 32 units were also placed at locations moved by ±60.5 mm, ±121 mm, ±181.5 mm, and ±242 mm in the z-axis direction, respectively.

Furthermore, space other than the aforementioned positron emission tomography device, the subject, and the like was assumed to be filled with the air.

Note that the size of each scintillator unit was assumed to be 60 mm in length, 60 mm in width, and L mm in thickness, and calculation was performed for each of L = 1, 2, 5, 10, and 20 mm. Further, the material of the scintillator was appropriately changed in Comparative Examples 1 and 2, and Example 1 to be described later, and the results were compared.

### <Emission Direction of Radiation and Inherent Background Radiation>

Calculation was performed assuming that all gamma rays emitted from the cancerous part were emitted in the x-axis direction. In Examples, all of the radiation source, the subject, and the scintillator are placed in a circularly symmetrical manner around the z-axis, and therefore even when the direction of emitted gamma rays is limited to one direction as described above, information equivalent to a case of gamma rays actually emitted in random directions is acquired from the acquired result, the information including efficiency of radiation detection and the like.

Inherent background radiation intensity was set for each material of the scintillator, and inherent background radiation was assumed to be generated from a scintillator unit that should detect a gamma ray or all scintillator units.

### <Radiation Detection Efficiency Related to Simultaneous Measurement and Method for Calculating S/N Value>

Based on the aforementioned condition, a situation in which emitted a gamma ray and background radiation are absorbed, scattered, or penetrated in the subject, the air, and the scintillator was simulated, and the number of gamma rays finally detected as signals was found after further evaluating the probability of detection in the scintillator for each energy value possessed by radiation.

Note that incident radiations are not necessarily detected in whole and are classified into detected radiations and undetected radiations as a result of an interaction between the radiation and the scintillator. The interaction is calculated by the Monte Carlo method, based on the program code GEANT4, with energy of radiation, and the density and the effective atomic number of the scintillator as parameters, and as a result, detection efficiency is derived for each energy value of radiation.

Next, the detected radiations were classified into the following three categories, based on energy value information:
1. a signal (S) related to a radiation to be recognized as a signal, that is, single measurement,
2. a noise (N) related to single counting that is detected because of possession of energy in the energy window but should not be recognized as a signal and should be employed as an N-value in S/N value calculation, and
3. a noise that is related to an energy band of background radiation or the like and therefore is not counted as an N-value in S/N value calculation; and detection efficiency was calculated based on the S-value, and the S/N value was calculated based on the S-value and the N-value.

Specifically, when one hundred thousand gamma rays possessing energy of 511 keV were emitted from the central part of the cancerous part, the number of gamma rays detected by a scintillator unit placed in the emission direction (the x-axis direction from the central part) of the gamma rays (may also be hereinafter described as a "scintillator unit that should detect a gamma ray"), that is, S- and N-values related to single measurement was measured. Next, [S/100000]^² was calculated as detection efficiency related to simultaneous measurement. Furthermore, denoting the number of signals related to simultaneous measurement by S^²/100000 and the number of noises related to simultaneous measurement by N, the S/N value was calculated by dividing the number of signals related to simultaneous measurement by the number of noises related to simultaneous measurement.

As for N, when a noise is detected in one scintillator unit, a signal or a noise is assumed to be actually detected in a pairing scintillator unit, and the same value is assumed for N related to single measurement and simultaneous measurement. Note that the condition for distinguishing between a signal and a noise, such as the energy window, was individually set in Comparative Example 1 and the Example 1 to be described later, and in some cases, a gamma ray detected by every scintillator unit other than the scintillator unit that should detect a gamma ray was also considered as a noise.

### <Comparative Example 1>

A simulation using a material similar to lutetium orthosilicate (LSO) as the scintillator was performed. Specifically, the effective atomic number and the density of the scintillator were set to 64 and 7.4 g/cm³, respectively. Note that the effective atomic number of LSO is calculated to be 64, based on Medical Physics, 39 (2012), p. 1769.

### <Setting of Background Radiation>

The inherent background radiation intensity of the scintillator was assumed to be 300 Hz/cm³ over the entire energy band, based on a literature value related to the inherent background intensity of LSO (arXiv preprint arXiv: 1501.05372, 2015 - arxiv.org). LSO emits inherent background radiation originating from a radioisotope, and main components thereof are gamma rays with energy of 88, 202, and 307 keV, and a beta ray with energy of 596 keV as a maximum value. The components are simultaneously generated in a series of flows of decay of the radioisotope, and therefore for example, a gamma ray with energy of 509 keV being the sum of 202 and 307 keV are apparently detected.

While inherent background further includes continuous components not possessing a peak at specific energy due to Compton components of the beta ray and the gamma ray, the components have a very low probability of occurrence (frequency) and therefore are not considered in the simulation. While inherent background is actually generated from all scintillator units, background radiation was set to be generated only from the scintillator unit that should detect a gamma ray in Comparative Example 1 for simplification. Further, while a component originating from environmental background generated by an environment in the device, a component generated by two radiations generated at separate locations randomly and simultaneously entering different scintillators, and the like may exist as background, the components also have a very low probability of occurrence (frequency) and therefore are not considered in the simulation.

### <Calculation of Detection Efficiency and S/N Value>

First, calculation was performed by a single measurement technique under a condition simulating a conventional signal processing method. Specifically, the energy resolution (FWHM value) was set to 51 keV, and the energy window was set to 511±102 keV. A gamma ray being detected by the scintillator unit that should detect a gamma ray and possessing an energy value within the energy window were counted in the number of events employed for calculation of detection efficiency and the S/N value.

Next, an event related to a gamma ray with energy of 511 keV out of the events was assumed to be a signal (S) related to single measurement, and every event related to a gamma ray being within the energy window and possessing an energy value other than 511 keV was assumed to be a noise (N) considered in S/N value calculation.

Finally, [S/100000]^² and (S^²/100000)/N were calculated as detection efficiency related to simultaneous measurement and an S/N value related to simultaneous measurement, respectively.

While an actual PET device based on a conventional method can use only a gamma ray with energy of 511 keV as a signal in principle, and a gamma ray with another energy value should be processed as a noise, the device on the other hand cannot distinguish a gamma ray with energy of 511 keV from a gamma ray with another energy value in an energy band (energy window) centered on 511 keV within a width of several times, such as four times, as much as energy resolution (an FWHM value, such as 50 keV corresponding to about 10% of 511 keV).

Therefore, in the actual PET device based on the conventional method, the number of gamma rays apparently detected as gamma rays with energy of 511 keV, that is, an apparent number of events is actually highly likely to include a noise in the energy window.

An energy value can be identified in units of 1 keV in the simulation, and therefore all gamma rays with energy values other than 511 keV that should be intrinsically processed as noises in the energy window were processed as noises.

The condition is described in Table 1, and the result is described in Table 2 and Table 3.

### <Comparative Example 2>

Detection efficiency and an S/N value that are related to simultaneous measurement were calculated similarly to Comparative Example 1 except that a scintillator material was a target material with an effective atomic number of 64 equivalent to LSO and a density of 8.1 g/cm³ and that inherent background was assumed to be negligible in the material. The condition is described in Table 1, and the result is described in Table 2 and Table 3.

### <Example 1>

Detection efficiency and an S/N value that are related to simultaneous measurement were calculated similarly to Comparative Example 2 except for the following changes. Specifically, in S/N value calculation, all electric signal groups with energy values within a range equal to or greater than 250 keV out of detected gamma rays were assumed to be gamma rays usable as signals. Next, as for gamma rays with energy equal to or greater than 232 keV and less than 250 keV out of the detected gamma rays, half the number of events detected by the scintillator unit that should detect a gamma ray were counted as signals (S) in single measurement, and the other half were counted as noises (N) used for S/N value calculation. Furthermore, as for a gamma ray detected by a scintillator unit other than the scintillator unit that should detect a gamma ray, all gamma rays with energy equal to or greater than 232 keV were counted as noises (N) used for S/N value calculation.

Note that use of all electric signal groups corresponding to gamma rays with an energy value equal to or greater than 250 keV as signals as described above reflects that, when radiation being energy less than 511 keV and possessing an energy value belonging to an energy band excluding relatively low-energy background radiation, such as back scattering, is detected in a case of the fluorescence lifetime of a scintillator material being short, thereby enabling shortening of the time window, and inherent background being negligible, the radiation can also be calculated as a true coincidence while only a gamma ray at 511 keV is calculated as a true coincidence in a conventional PET method.

Further, handling of the aforementioned gamma rays with energy equal to or greater than 232 keV and less than 250 keV takes into account that about half of the gamma rays may be detected as gamma rays with energy equal to or greater than 250 keV in an actual device due to energy resolution.

Note that when the range of N in Comparative Examples 1 and 2 based on a conventional method is set similarly to the range of N in Example 1, that is, when an S/N value is determined by a method similar to that in Comparative Examples 1 and 2 but the range of the energy window determining a noise is widened to a processing target range (equal to or greater than 232 keV) in Example 1, both detection efficiency and an S/N value become very low, and therefore the above is not applicable to the positron emission tomography device.

The condition of the simulation is described in Table 1, and the result of the simulation is described in Table 2 and Table 3.

### [Table 1]

**Table 1 Simulation conditions**

| | Scintillator | | | | Energy window /keV | Count noise originating from every scintillator unit |
|---|---|---|---|---|---|---|
| | Effective atomic number | Density /gcm⁻³ | Thickness L /mm | Inherent background radiation intensity /Hz/cc | | |
| Comparative Example 1-1 | 64 | 7.4 | 1 | 300 | 409~613 | No |
| Comparative Example 1-2 | 64 | 7.4 | 2 | 300 | 409~613 | No |
| Comparative Example 1-3 | 64 | 7.4 | 5 | 300 | 409~613 | No |
| Comparative Example 1-4 | 64 | 7.4 | 10 | 300 | 409~613 | No |
| Comparative Example 1-5 | 64 | 7.4 | 20 | 300 | 409~613 | No |
| Comparative Example 2-1 | 64 | 8.1 | 1 | 0 | 409~613 | No |
| Comparative Example 2-2 | 64 | 8.1 | 2 | 0 | 409~613 | No |
| Comparative Example 2-3 | 64 | 8.1 | 5 | 0 | 409~613 | No |
| Comparative Example 2-4 | 64 | 8.1 | 10 | 0 | 409~613 | No |
| Comparative Example 2-5 | 64 | 8.1 | 20 | 0 | 409~613 | No |
| Example 1-1 | 64 | 8.1 | 1 | 0 | 250~613 | Yes |
| Example 1-2 | 64 | 8.1 | 2 | 0 | 250~613 | Yes |
| Example 1-3 | 64 | 8.1 | 5 | 0 | 250~613 | Yes |
| Example 1-4 | 64 | 8.1 | 10 | 0 | 250~613 | Yes |
| Example 1-5 | 64 | 8.1 | 20 | 0 | 250~613 | Yes |

### [Table 2]

**Table 2 Detection efficiency related to simultaneous measurement**

| Scintillator thickness L /mm | Detection efficiency /% | | |
|---|---|---|---|
| | Comparative Examples 1-1 to 1-5 | Comparative Examples 2-1 to 2-5 | Examples 1-1 to 1-5 |
| 1 | 0.0148 | 0.0181 | 0.0509 |
| 2 | 0.100 | 0.112 | 0.223 |
| 5 | 0.887 | 0.945 | 1.41 |
| 10 | 3.470 | 3.64 | 4.78 |
| 20 | 9.78 | 10.1 | 12.4 |

### [Table 3]

**Table 3 S/N value**

| Scintillator thickness L /mm | S/N value | | |
|---|---|---|---|
| | Comparative Examples 1-1 to 1-5 | Comparative Examples 2-1 to 2-5 | Examples 1-1 to 1-5 |
| 1 | 0.0210 | 0.0804 | 0.303 |
| 2 | 0.0836 | 0.308 | 0.751 |
| 5 | 0.332 | 1.28 | 2.82 |
| 10 | 0.719 | 3.06 | 7.80 |
| 20 | 1.10 | 5.69 | 19.9 |

In any of aforementioned Example 1 and Comparative Examples 1 and 2, energy of radiation taking on, out of detected signal values, a signal value when a gamma ray with energy of 511 keV entered the scintillator (scintillator unit) and was totally absorbed by the scintillator was 511 keV. Accordingly, in the simulations in Comparative Examples 1 and 2 set to the aforementioned conditions, only a signal value with the ratio of a minimum signal value in a processing target signal to the signal value when a gamma ray with energy of 511 keV in a processing target signal entered the scintillator and was totally absorbed by the scintillator being 100% was recognized as a processing target. On the other hand, in the simulation in Example 1 set to the aforementioned condition, the ratio of a minimum signal value in a processing target signal to the signal value when a gamma ray with energy of 511 keV in the processing target signal entered the scintillator and was totally absorbed by the scintillator at 232 to 511 keV was 45%. Accordingly, in the simulation in Example 1, every signal value with the ratio of a minimum signal value in a processing target signal to the signal value when a gamma ray with energy of 511 keV in the processing target signal entered the scintillator and was totally absorbed by the scintillator is equal to or greater than 45% was recognized as a processing target.

As indicated in the tables, the positron emission tomography device in Example 1 exhibited high detection efficiency related to simultaneous measurement. Further, with regard to an S/N value, Example 1 exhibited a high value in spite of a condition more stringent than Comparative Example 1 and Comparative Example 2 in counting a noise in every scintillator unit.

As for an S/N value in particular, as can be understood by comparing Comparative Example 2 with Example 1, a twice or three times as much S/N value was exhibited with the same scintillator thickness.

Further, as can be understood by comparing Comparative Example 1 with Example 1, by using a scintillator material with low inherent background intensity in addition to changing the detection method, the S/N value was further improved, and a greater than eight times as much S/N value or a close to 20 times as much value depending on the thickness was exhibited with the same scintillator thickness, compared with a case of using the conventional method and the conventional material.

As described above, the present invention can provide a signal processing system, a positron emission tomography device, and a positron emission tomography method that improve detection efficiency and/or an S/N value compared with conventional systems, devices, and methods.

### DESCRIPTION OF SYMBOLS

- 1:: subject
- 2:: scintillator
- 3:: cancerous part

## Claims

1. A signal processing system generating image data, based on an electric signal group output from a radiation detector,
wherein the signal processing system recognizes the electric signal group as a processing target, and
the electric signal group includes at least part of an electric signal group meeting requirements described below:
the electric signal group is an electric signal group with a signal value within a predetermined range, the electric signal group corresponding to a gamma ray with energy equal to or less than 375 keV;
the predetermined range is equal to or greater than 50% and equal to or less than 80% relative to a 100% signal value; and
the 100% signal value is a signal value detected when a gamma ray with energy of 511 keV enters a radiation detection element in a radiation detector and is totally absorbed by the radiation detection element.

2. A signal processing system generating image data, based on an electric signal group output from a radiation detector,
wherein the signal processing system recognizes the electric signal group as a processing target, and
the electric signal group includes at least part of an electric signal group corresponding to a gamma ray with an energy value within a predetermined range, and the predetermined range is equal to or greater than 232 keV and equal to or less than 340 keV.

3. A positron emission tomography device comprising the signal processing system according to claim 1 or 2 and a radiation detector section.

4. The positron emission tomography device according to claim 3, wherein the radiation detector section includes components described below:
a scintillator section including a scintillator receiving radiation and emitting an electromagnetic wave; and a conversion-output section receiving an electromagnetic wave emitted from the scintillator, converting the received electromagnetic wave into a pulse-shaped electric signal, and outputting the resulting signal.

5. The positron emission tomography device according to claim 4, wherein the scintillator meets a characteristic described below:
intensity of inherent background of the scintillator is equal to or less than 200 Hz/cm³ in a range of a signal value equal to or greater than 10% and equal to or less than 120% with a signal value of the pulse-shaped electric signal when a gamma ray with energy of 511 keV enters the scintillator and is totally absorbed by the scintillator as 100%.

6. The positron emission tomography device according to claim 4 or 5, wherein a time window in the conversion-output section is equal to or less than 180 ns.

7. The positron emission tomography device according to any one of claims 4 to 6, wherein a fluorescence decay time (DT) of the scintillator when the scintillator is irradiated with a gamma ray is equal to or less than 25 ns.

8. The positron emission tomography device according to any one of claims 4 to 7, wherein a gamma-ray absorption coefficient of the scintillator is equal to or greater than 70%.

9. The positron emission tomography device according to any one of claims 4 to 7, wherein a gamma-ray absorption coefficient of the scintillator is equal to or less than 50%.

10. A signal processing method comprising generating image data, based on an electric signal group output from a radiation detector,
wherein the signal processing method recognizes the electric signal group as a processing target, and
the electric signal group includes at least part of an electric signal group meeting requirements described below:
the electric signal group is an electric signal group with a signal value within a predetermined range, the electric signal group corresponding to a gamma ray with energy equal to or less than 375 keV;
the predetermined range is equal to or greater than 50% and equal to or less than 80% relative to a 100% signal value; and
the 100% signal value is a signal value detected when a gamma ray with energy of 511 keV enters a radiation detection element in a radiation detector and is totally absorbed by the radiation detection element.

11. A signal processing method comprising generating image data, based on an electric signal group output from a radiation detector,
wherein the signal processing method recognizes the electric signal group as a processing target, and
the electric signal group includes at least part of an electric signal group corresponding to a gamma ray with an energy value within a predetermined range, and the predetermined range is equal to or greater than 232 keV and equal to or less than 340 keV.

12. A positron emission tomography method comprising at least steps (a), (b), and (c) described below:
(a) a scintillation step of converting radiation into an electromagnetic wave by using a scintillator receiving radiation and emitting an electromagnetic wave;
(b) a conversion-output step of receiving an electromagnetic wave emitted from the scintillator, converting the received electromagnetic wave into a pulse-shaped electric signal, and outputting the resulting signal; and
(c) a signal processing step including a step of performing signal processing by the signal processing method according to claim 10 or 11.

13. The positron emission tomography method according to claim 12, wherein the scintillator meets a characteristic described below:
intensity of inherent background of a scintillator is equal to or less than 200 Hz/cm³ in a range of a signal value being 10 to 120% with a signal value of the pulse-shaped electric signal when a gamma ray with energy of 511 keV enters the scintillator and is totally absorbed by the scintillator as 100%.

14. The positron emission tomography method according to claim 12 or 13, wherein a time window in the conversion-output section is equal to or less than 180 ns.

15. The positron emission tomography method according to any one of claims 12 to 14, wherein a fluorescence decay time (DT) of the scintillator when the scintillator is irradiated with a gamma ray is equal to or less than 25 ns.

16. The positron emission tomography method according to any one of claims 12 to 15, wherein a gamma-ray absorption coefficient of the scintillator is equal to or greater than 70%.

17. The positron emission tomography method according to any one of claims 12 to 15, wherein a gamma-ray absorption coefficient of the scintillator is equal to or less than 50%.
